# EUROPÄISCHE PATENTSCHRIFT

(11) **EP 1 051 621 B1**
(45) Veröffentlichungstag und Bekanntmachung des Hinweises auf die Patenterteilung: **20.04.2005**
(21) Anmeldenummer: 99907282.0
(22) Anmeldetag: 23.01.1999
(51) Int. Cl.: G01N 33/543, G01N 33/58, G01N 27/327

(54) **VERFAHREN ZUR ANALYSE EINER PROBE MITTELS EINES ELEKTROCHEMOLUMINESZENZ-BINDUNGSREAKTION-TESTS**
METHOD FOR ANALYZING A TEST SAMPLE BY MEANS OF AN ELECTRO-CHEMOLUMINESCENCE BOND REACTION TEST
PROCEDE D'ANALYSE D'UN ECHANTILLON SELON UN ESSAI DE REACTION DE LIAISON FAISANT APPEL A L'ELECTROCHIMIOLUMINESCENCE

(30) Priorität: 30.01.1998 DE 19803528
(43) Veröffentlichungstag der Anmeldung: 15.11.2000
(73) Patentinhaber: Roche Diagnostics GmbH, 68298 Mannheim (DE)
(72) Erfinder: EGGER, Martin, D-82347 Bernried (DE); PUNZMANN, Gabriele, D-81547 München (DE); MÜLLER, Günter, D-82380 Peissenberg (DE); PAUSELIUS-FUCHS, Ursula, D-82319 Starnberg (DE)
(74) Vertreter: Pfeifer, Hans-Peter, Dr.
(86) Internationale Anmeldenummer: PCT/DE1999/000197
(87) Internationale Veröffentlichungsnummer: WO 1999/039206

(56) Entgegenhaltungen:
- EP-A- 0 647 849
- WO-A-89/10551
- WO-A-90/11511
- WO-A-92/14138

## Beschreibung

Die Erfindung betrifft ein Verfahren zur Analyse einer Probe bezüglich einer darin befindlichen Substanz.

Bei der Analyse einer flüssigen Probe geht es in der Regel darum, die Konzentration einer darin enthaltenen Substanz (Analyt) zu bestimmen (quantitative Analyse). In manchen Fällen genügt auch eine Aussage, ob der Analyt (in einer-oberhalb eines Grenzwerts liegenden Konzentration) in der Probe enthaltenen ist (qualitative Analyse). Auf medizinischem Gebiet, auf das sich die Erfindung insbesondere bezieht, spielt die Analyse von Körperflüssigkeiten (vor allem Blut, Blutserum und Urin) auf darin enthaltene Analyten wie zum Beispiel Hormone, Antikörper, Antigene oder Medikamente eine große Rolle.

Die Erfindung befaßt sich mit der Verbesserung eines bestimmten Typs von Analyseverfahren, den man zusammenfassend als Elektrochemolumineszenz-Bindungsreaktion-Analyse (nachfolgend als "ECL-BBA" für electrochemoluminescence biochemical binding analysis bezeichnet) bezeichnen kann. Ein solches Verfahren weist folgende charakteristische Merkmale auf.
a) Grundlage der analytischen Selektivität ist eine spezifische biochemische Bindungsreaktion unter Beteiligung biochemischer Substanzen, die nur miteinander selektiv bindungsfähig sind. Hierzu gehören vor allem immunchemische Bindungsreaktionen zwischen Antikörpern und Antigenen oder Haptenen, mit denen die Antikörper spezifisch binden. Andere biochemische Bindungsreaktionen sind Proteinbindungen, insbesondere zwischen Avidin und Biotin, die Lektin-Kohlenhydrat-Bindung, die Bindung zwischen Rezeptoren und Liganden und die Hybridisierung von Nukleinsäuren.
   Solche spezifischen biochemischen Bindungsreaktionen werden seit langem für analytische Zwecke verwendet. Es gibt zahlreiche unterschiedliche ein- oder mehrstufige Reaktionsabläufe ("Testprotokolle"), die letztlich unter Beteiligung des Analyten und mindestens einer in dem Reagenzsystem enthaltenen spezifisch bindenden Substanz ("Bindungsreagenz") zur Bildung eines für die Analyse charakteristischen Komplexes führen. Dieser Komplex enthält in der Regel (jedoch nicht notwendigerweise) den Analyten.
b) Um den Komplex, dessen Konzentration ein Maß für das gesuchte analytische Resultat bildet, detektierbar zu machen, wird in der Regel eine Markersubstanz ("Label") eingesetzt, die an ein Bindungsreagenz des Reagenzsystems, beispielsweise einen Antikörper, gekoppelt wird. Die Einheit aus Markersubstanz und Bindungsreagenz wird als Konjugat bezeichnet.
   Die Erfindung bezieht sich auf Verfahren, bei denen die Markersubstanz zu einer ECL-Reaktion fähig ist. Wenn eine solche Substanz an einer voltametrischen Elektrode einem geeigneten elektrischen Potential ausgesetzt wird, sendet sie Licht aus, das photometrisch gemessen werden kann. In der Regel ist an dieser Reaktion eine zweite elektrochemisch aktive Substanz beteiligt, die als "Precursor" bezeichnet wird. In der Praxis wird als ECL-Label vor allem ein Rutheniumkomplex (Ruthenium-tris[bipyridyl]), in Kombination mit TPA (Tripropylamin) als Precursor, verwendet. Die beiden elektrochemisch aktiven Substanzen reagieren an der Elektrode jeweils unter Abgabe eines Elektrons zu einer stark reduzierenden bzw. oxidierenden Spezies. Die nachfolgende Redoxreaktion bringt das ECL-Label in einen angeregten Zustand, von dem es unter Aussendung eines Photons in den Grundzustand zurückfällt. Die Reaktion des ECL-Labels ist vorzugsweise eine Kreisreaktion, so daß ein einziges Label-Molekül nach Anlegen einer Spannung an die Elektrode eine Vielzahl von Photonen aussendet.
c) Bei den Verfahren, auf die sich die Erfindung bezieht, werden die ECL-markierten für die Analyse charakteristischen Komplex-Moleküle an magnetische Mikropartikel ("Beads") fixiert. In der Praxis werden magnetisierte Polystyrolkügelchen mit einem Durchmesser von typischerweise etwa 2 bis 3 µm verwendet. Die Fixierung erfolgt mittels eines Paares spezifischer biochemischer Bindungspartner, wobei sich insbesondere das Paar Streptavidin-Biotin bewährt hat. Die Beads sind mit Streptavidin-Polymer beschichtet. An die Komplex-Moleküle ist Biotin gebunden.
   Die Beads mit dem daran gebundenen markierten Komplex werden in die Meßzelle eines Meßgerätes gebracht, das die erforderlichen Elektroden (in der Regel eine Arbeitselektrode, eine Gegenelektrode und, insbesondere im Fall eines potentiometrischen Meßprinzips, eine Referenzelektrode) zur Erzeugung des zum Triggern der ECL-Reaktion erforderlichen elektrischen Feldes aufweist. Im magnetischen Feld eines unter der Arbeitselektrode angeordneten Magneten werden die Beads an die Oberfläche der Arbeitselektrode gezogen. Da dies üblicherweise in Durchflußzellen bei kontinuierlich strömender Probenflüssigkeit stattfindet, wird die magnetisch bewirkte Ablagerung der Beads als "Abfangen" bezeichnet.
   Nach dem Abfangschritt wird in der Regel ein Waschschritt durchgeführt, bei dem eine Waschflüssigkeit an der Arbeitselektrode vorbeiströmt und störende Komponenten entfernt. Danach wird ein zum Triggern der ECL-Reaktion erforderliches elektrisches Potential an die Arbeitselektrode angelegt und das resultierende Lumineszenz-Licht mittels eines geeigneten Fotoempfängers gemessen. Die Intensität des Lumineszenzlichts ist ein Maß für die Konzentration der markierten Beads an der Oberfläche der Arbeitselektrode und diese wiederum ist ein Maß für die Konzentration des Analyten in der Probe. Zur Berechnung der gesuchten Konzentration aus dem gemessenen Lumineszenzsignal dient eine Kalibration.

Es werden zahlreiche unterschiedliche Varianten derartiger ECL-BBA-Verfahren diskutiert und in der Literatur beschrieben, wobei sich die Abwandlungen auf jeden der genannten Teilaspekte beziehen können.

Hinsichtlich des Teilaspektes a) unterscheiden sich die Tests durch verschiedene Testprotokolle (beispielsweise Sandwich-Tests und kompetitive Tests, jeweils in einer Vielzahl möglicher Untervarianten). Ein grundsätzlicher Unterschied besteht zwischen homogenen Tests, die keine Trennung zwischen den gebildeten Komplex-Molekülen und unkomplexiertem Konjugat erfordern und heterogenen Tests, bei denen eine solche bound/free-Trennung notwendig ist. Die vorliegende Erfindung ist für die unterschiedlichsten Testprotokolle anwendbar, soweit in irgendeiner Weise eine Reaktionsfolge abläuft, die mindestens eine spezifische biochemische Bindungsreaktion einschließt und zur Bildung eines für die Analyse charakteristischen Komplexes führt, der mit einem ECL-Label markiert ist.

Auch hinsichtlich des Teilaspektes b) ist die Erfindung universell, d.h. unabhängig von dem verwendeten ECL-Label und eventuellen weiteren Komponenten der ECL-Reaktion, einsetzbar. Sie hat sich insbesondere für Tests bewährt, bei denen der erwähnte Rutheniumkomplex in Verbindung mit TPA verwendet wird.

Hinsichtlich des Teilaspektes c) richtet sich die Erfindung ausschließlich auf Tests, bei denen der für die Analyse charakteristische Komplex an magnetische Mikropartikel gebunden wird und diese Mikropartikel im Magnetfeld eines Magneten auf der Oberfläche einer Arbeitselektrode abgelagert werden. Im übrigen ist die Erfindung auch von Variationen des Teilaspekts c) unabhängig und beispielsweise mit unterschiedlichen Bead-Materialien und -Größen sowie mit unterschiedlichen Methoden zur Fixierung der Komplexe an den Beads verwendbar.

Nähere Informationen über ECL-BBA-Verfahren können der einschlägigen Literatur entnommen werden. Hierzu wird insbesondere auf folgende Publikationen verwiesen, deren Inhalt durch Bezugnahme zum Bestandteil der vorliegenden Beschreibung gemacht wird:
1) G.F. Blackburn et al. "Electrochemiluminescence Detection for Development of Immunoassays and DNA Probe Assays for Clinical Diagnostics", Clin.Chem. 37 (1991), 1534 - 1539
2) J.K. Leland und M.J Powell: "Electrogenerated Chemiluminescence: An Oxidative-Reduction Type ECL Reaction Sequence Using Tripropyl Amine", J. Electrochem. Soc., 137 (1990), 3127 - 3131
3) J.H. Kenten et al.: "Improved Electrochemiluminescent Label for DNA Probe Assays: Rapid Quantitative Assays of HIV-1 Polymerase Chain Reaction Products", Clin.Chem. 38 (1992), 873 - 879
4) N.R. Hoyle: "The Application of Electrochemiluminescence to Immunoassay-based Analyte Measurement" in "Bioluminescence and Chemiluminescence"; Proceedings of the 8th International Symposium on Bioluminescence and Chemiluminescence, Cambridge, September 1994, A.K. Campbell et al. (edit.), John Wiley & Sons
5) WO 89/10551
6) WO 90/11511

Wie erwähnt findet die Messung des ECL-Lichtes üblicherweise in einer Durchflußmeßzelle statt. Sie enthält einen engen Strömungskanal für die Probenflüssigkeit, wobei die Arbeitselektrode an einer der Wände, die den Strömungskanal begrenzen, angeordnet ist. Um mit der gleichen Meßzelle nacheinander unterschiedliche Proben messen zu können, muß die Zelle, und insbesondere die Arbeitselektrode, zwischen den Messungen von den darauf abgelagerten Beads und sonstigen Verunreinigungen gereinigt werden. Dieser Reinigungsvorgang muß schnell und effizient sein, um eine hohe Durchsatzleistung des Analysegeräts und eine gute Analysegenauigkeit sicherzustellen.

Die Reinigung erfolgt deswegen nicht nur physikalisch (Durchleiten von Luftblasen) und chemisch (Durchleiten einer Reinigungsflüssigkeit, die unter anderem Detergenzien enthält), sondern auch elektrochemisch durch Anlegen eines stark oxidierenden und/oder reduzierenden Potentials an die Arbeitselektrode. Das Potential ist in der Regel so hoch, daß an der Oberfläche der Arbeitselektrode Gasblasen entstehen. Dadurch wird die Reinigung wirksam unterstützt, das elektrochemische Gleichgewicht der Elektrodenoberfläche jedoch so stark gestört, daß im Anschluß an den Reinigungsschritt ein Konditionierungsschritt erforderlich ist, bei dem eine Sequenz von Pulsen an die Arbeitselektrode angelegt wird, die den gesamten Potentialarbeitsbereich des jeweiligen Elektrodenmaterials abdecken.

In der Zelle wird demzufolge ein Detektionszyklus durchgeführt, der nacheinander einen Reinigungsschritt, einen Konditionierungsschritt, einen Abfangschritt und einen Meßschritt umfaßt. Während des Reinigungsschritts und des Konditionierungsschritts befindet sich eine Reinigungs- bzw. Konditionierungsflüssigkeit in der Zelle. Die Probenflüssigkeit mit den Beads wird erst zu Beginn des Abfangschritts in die Durchflußmeßzelle eingeleitet. Bei heterogenen Tests ist zwischen Abfangschritt und Meßschritt ein zusätzlicher Waschschritt eingefügt. Der Detektionszyklus wird in den Zitaten 1) bis 6), vor allem in der WO 89/10551, näher erläutert.

Das ECL-BBA-Verfahren zeichnet sich im Vergleich zu anderen Analyseverfahren, die auf der spezifischen Bindung biochemischer Bindungspartner beruhen, durch einfache Handhabung, hohe Empfindlichkeit, einen großen dynamischen Bereich meßbarer Konzentrationen, eine kostengünstige Analyse und gute Automatisierbarkeit (mit entsprechenden Analysegeräten) aus.

Um eine weitere Erhöhung der Analysegenauigkeit zu erreichen, wird ein Verfahren gemäß Anspruch 1 vorgeschlagen.

Der Zusammenhang zwischen einem während des Detektionszyklus an der Arbeitselektrode anliegenden Spannungsverlauf und der Qualität der Analyse wird in der WO 89/10551 (Zitat 5) diskutiert. Danach soll zur Verbesserung der Reproduzierbarkeit des Analyseergebnisses am Ende des Konditionierungsschritts ein konstanter Potentialwert eingestellt werden, der als "preoperative potential" bezeichnet wird. Dieses "preoperative potential" soll konstant bleiben, bis die Arbeitselektrode mit der Probenflüssigkeit kontaktiert und die ECL-Messung durchgeführt wird. Das "preoperative potential" soll in Abhängigkeit von dem Material der Arbeitselektrode und in Abhängigkeit von dem verwendeten Elektrolyten entweder ein oxidierendes oder ein reduzierendes Potential sein.

Im Rahmen der Erfindung wurde festgestellt, daß im Gegensatz zu der Lehre des Zitats 5) eine wesentliche Verbesserung der gleichmäßigen Ablagerung der Beads auf der Oberfläche der Arbeitselektrode und damit eine Verbesserung der Reproduzierbarkeit und Genauigkeit der Analyse erreicht werden kann, wenn in den Spannungsverlauf des Detektionszyklus der zusätzliche Potentialpuls eingefügt ist. Wesentlich ist, daß dieser einerseits ein oxidierendes oder reduzierendes Potential erreicht und andererseits auf ein neutrales (weder oxidierendes noch reduzierendes) Potential zurückgeführt wird, bevor die Probe in die Meßzelle eingeleitet und somit in Kontakt zu der Arbeitselektrode gebracht wird.

Während durch das "preoperative potential" der WO 89/10551 die für die Erzeugung des ECL-Signals entscheidenden Komponenten der Probe beeinflußt werden sollen, wird erfindungsgemäß eine erhebliche Verbesserung durch eine zusätzliche elektrochemische Vorbehandlung der Elektrode erreicht. Daß daraus eine Verbesserung der Ablagerung der Beads resultiert, ist unerwartet, weil angenommen werden mußte, daß die Bead-Verteilung von den Eigenschaften des magnetischen Feldes abhängt, während die elektrochemischen Maßnahmen der Konditionierung und Reinigung dem Zweck dienten, die Signalerzeugung zu verbessern.

Ein Potentialpuls in diesem Sinne ist eine kurzzeitige Änderung der an der Arbeitselektrode anliegenden Spannung, während der ein oxidierender bzw. reduzierender Potentialwert erreicht wird, wobei sich die elektrische Spannung vor und nach dem Potentialpuls im neutralen Bereich befindet. Auf die Form des Potentialverlaufs im einzelnen kommt es nicht an. Es ist also insbesondere nicht erforderlich, daß der Spannungsverlauf eine geometrisch definierte Form (z.B. Rechteck, Dreieck oder Treppenfunktion) hat.

Als oxidierendes Potential wird ein elektrisches Potential der Arbeitselektrode bezeichnet, bei dem deren Oberfläche in Kontakt mit der Konditionierungsflüssigkeit oxidiert wird. Entsprechend ist ein Potential reduzierend, wenn es eine elektrochemische Reduktion der Metalloberfläche der Arbeitselektrode in Kontakt mit der Konditionierungsflüssigkeit bewirkt. Neutral ist ein Potential, bei dem auf einer blanken Metalloberfläche praktisch keine Oxid- oder Hydrid-Schicht gebildet wird.

Dieser Zustand wird auch als Doppelschichtbereich des jeweiligen Metalles bezeichnet.

Allgemein gültige Zahlenwerte für das maximale Potential des zusätzlichen Pulses und für den Potentialwert, auf den dessen Potential zurückgeführt wird, können nicht angegeben werden, weil diese Potentialwerte von dem Material der Arbeitselektrode, von der Referenzelektrode, auf die, sich das Potential bezieht und (in geringerem Umfang) von der Zusammensetzung der Konditionierungsflüssigkeit abhängig sind. Der Fachmann kann hierzu nähere Informationen aus veröffentlichten Daten, insbesondere aus Cyclovoltamogrammen des verwendeten Elektrodenmaterials, entnehmen. In jedem Fall lassen sich die Werte eines oxidierenden, reduzierenden oder neutralen Potentials experimentell bestimmen.

Die Erfindung wird im folgenden anhand eines in den Figuren schematisch dargestellten Ausführungsbeispiels näher erläutert. Es zeigen:
- Fig. 1: eine schematische Darstellung der Detektionseinheit eines ECL-BBA-Analysegeräts,
- Fig. 2: eine graphische Darstellung des Potentialverlaufs an der Arbeitselektrode während eines Detektionszyklus,
- Fig. 3: ein Cyclovoltamogramm einer Platinelektrode.

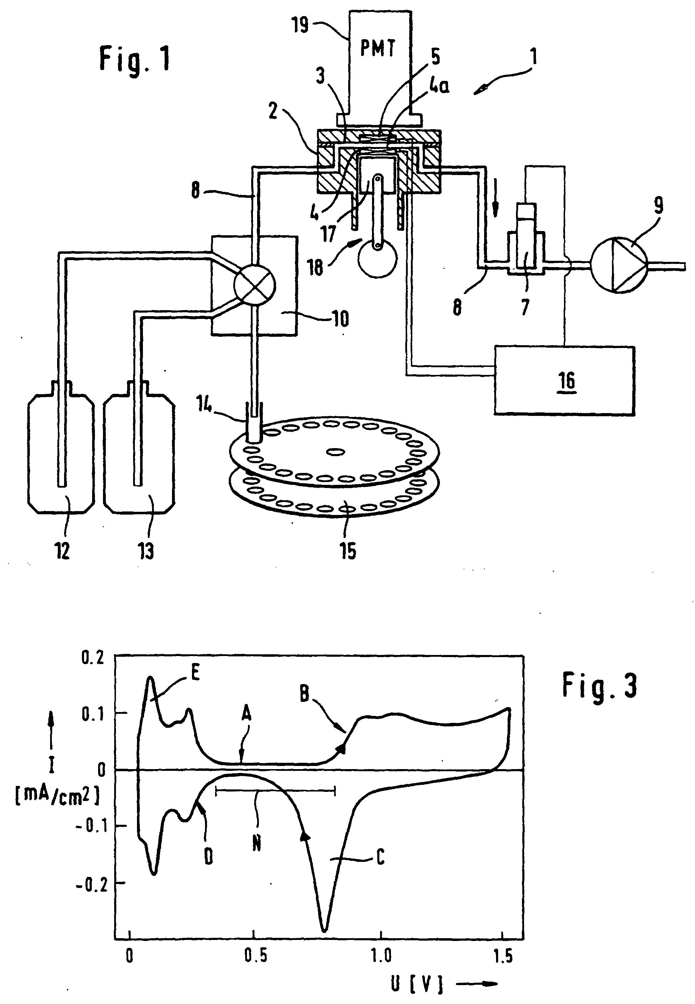

Die in Fig. 1 dargestellte Detektionseinheit 1 bildet denjenigen Teil eines Analysegeräts, der den Detektionszyklus automatisch durchführt. Daneben weist ein solches Analysegerät Einheiten zur Durchführung der Reaktionsfolge auf, die zur Bildung eines mit einem ECL-Label markierten Komplexes (dessen Konzentration für die Analyse charakteristisch ist) und zu dessen Bindung an magnetische Mikropartikel führt. Diese Teile sind in Fig. 1 nicht dargestellt.

Herzstück der Detektionseinheit 1 ist eine elektrochemische Durchflußzelle 2, in der an einem engen Durchflußkanal 3 eine Arbeitselektrode 4 und eine Gegenelektrode 5 derartig angeordnet sind, daß sie von einer durch den Durchflußkanal 3 strömenden Flüssigkeit kontaktiert werden. Die Gegenelektrode 5 ist bevorzugt, wie dargestellt, gegenüber der Arbeitselektrode 4 (d.h. auf der gegenüberliegenden Seite des Durchflußkanals 3) positioniert. Eine Referenzelektrode 7 ist, in der Regel außerhalb des Durchflußkanals 3, an der insgesamt mit 8 bezeichneten Flüssigkeitsleitung der Detektionseinheit 1 angeordnet. Zum Ansaugen der Flüssigkeit wird üblicherweise eine Präzisions-Kolbenhubpumpe 9 eingesetzt, die stromabwärts von der Durchflußzelle in die Flüssigkeitsleitung 8 eingebaut ist.

Stromaufwärts von der Durchflußzelle 2 sind an die Leitung 8 mehrere Flüssigkeitsbehälter angeschlossen, aus denen Flüssigkeit wahlweise, gesteuert beispielsweise durch ein Mehrwegventil 10, in die Durchflußzelle 2 gesaugt werden kann. Im dargestellten Fall sind dies ein Behälter 12 mit Reinigungsflüssigkeit, ein Behälter 13 mit Konditionierungsflüssigkeit und ein Behälter 14 mit Probenflüssigkeit. Der Probenflüssigkeitsbehälter 14 ist üblicherweise als Teströhrchen (reaction tube) ausgebildet und sitzt in einem Bearbeitungsrotor 15, in dem auch die erforderlichen Schritte zur Durchführung der Bindungsreaktionen stattfinden. Von den in dem Bearbeitungsrotor 15 sitzenden Teströhrchen ist der Übersichtlichkeit halber nur eines dargestellt.

In der Durchflußmeßzelle 2 ist auf der von dem Durchflußkanal 3 abgewandten Seite der Arbeitselektrode 4 ein Magnet 17 angeordnet. Im dargestellten Fall handelt es sich um einen Permanentmagnet, der mittels einer Bewegungsmechanik 18 von der dargestellten Abfangposition (bei der mindestens einer seiner Pole möglichst nah bei der Arbeitselektrode 4 ist) in eine von der Arbeitselektrode 4 entfernte Ruheposition beweglich ist. Alternativ kann auch ein ein- und ausschaltbarer Elektromagnet verwendet werden.

Auf der der Abfangfläche 4a gegenüberliegenden Seite des Durchflußkanals 3 ist als Lichtdetektor 19 ein Photomultiplier so positioniert, daß seine lichtempfindliche Fläche parallel zu der als Ablagerungsfläche 4a bezeichneten, dem Durchflußkanal 3 zugewandten (und von dem Magneten 17 abgewandten) Oberfläche der Arbeitselektrode 4 verläuft.

Zur Steuerung des Gerätes und Verarbeitung der Signale des Lichtdetektors 19 ist eine Elektronikeinheit 16 vorgesehen, an die die Elemente der Detektionseinheit 1 angeschlossen sind (Anschlußleitungen nur teilweise dargestellt).

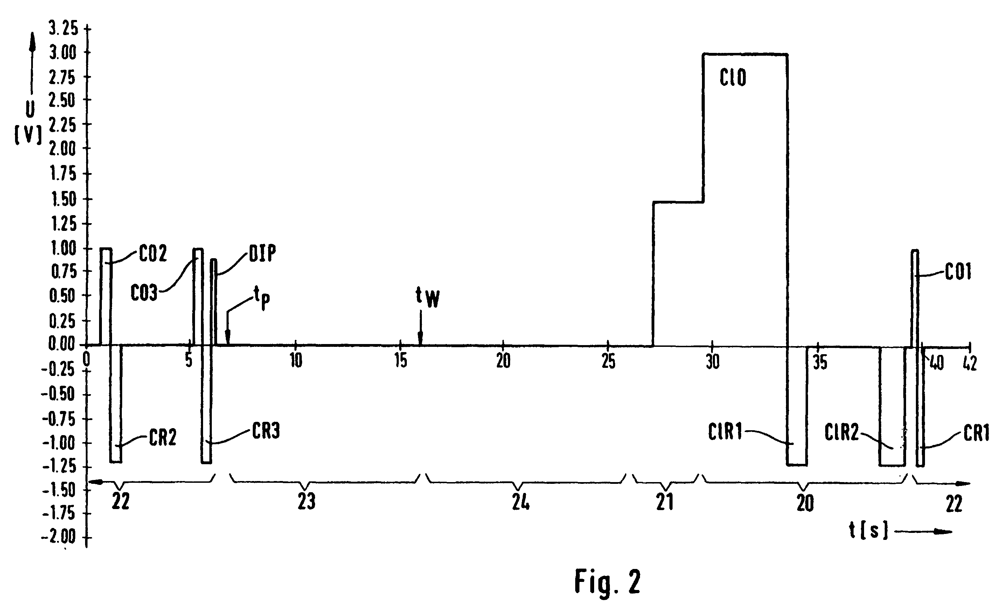

Für die Funktion der Detektionseinheit 1 ist wesentlich, daß während der einzelnen Verfahrensschritte eines Detektionszyklus der an der Arbeitselektrode 4 (bezogen auf die jeweils mit dieser in Kontakt stehende Flüssigkeit und somit bezogen auf die Referenzelektrode) anliegende Potentialverlauf bestimmte Merkmale aufweist, die nachfolgend anhand der graphischen Darstellung eines solchen Potentialverlaufes in Figur 2 näher erläutert werden. Die Figur bezieht sich auf eine Ausführungsform mit einer Platin-Arbeitselektrode. Die auf der Ordinate angegebenen Werte der Spannung U sind gegen eine Ag/AgCl-Referenzelektrode gemessen und gegen die Zeit t in Sekunden aufgetragen. Der Detektionszyklus wird für jede Analyse in gleicher Weise wiederholt. Die nachfolgende Beschreibung beginnt mit dem Reinigungsschritt.

Ein Reinigungsschritt 20 wird jeweils nach der vorausgehenden Messung durchgeführt, um die Ablagerungsfläche 4a der Arbeitselektrode 4 von darauf haftenden Beads und sonstigen Verunreinigungen oder Veränderungen der Elektrodenoberfläche zu befreien. Dabei wird zur elektrochemischen Unterstützung des Reinigungsvorganges ein stark oxidierendes und/oder reduzierendes Potential ClO bzw. C1R an die Arbeitselektrode angelegt, dessen Potentialwert in der Regel höher ist als jedes andere (oxidierende bzw. reduzierende) Potential des Detektionszyklus. Bevorzugt ist - wie dargestellt - ein oxidierendes Potential ClO, dessen Wert höher ist als das ebenfalls oxidierende Potential des vorausgehenden Meßschrittes 21. Im dargestellten Fall enthält der Reinigungsschritt zwei kleinere reduzierende Pulse C1R1 und C1R2. Dies ist jedoch nicht zwingend. Die Erfindung ist für jeden Detektionszyklus geeignet, bei dem während des Reinigungsschrittes ein oxidierendes oder reduzierendes Potential an die Arbeitselektrode angelegt wird, das so stark ist, daß nachfolgend zur Wiederherstellung des elektrochemischen Gleichgewichtes ein Konditionierungsschritt notwendig ist. Während des gesamten Reinigungsschrittes wird mittels der Pumpe 9 Reinigungsflüssigkeit aus dem Behälter 12 durch die Leitung 8 und somit auch durch den Durchflußkanal 3 geleitet.

Der nachfolgende Konditionierungsschritt 22 dient dazu, nach dem Reinigungsschritt 20 das erforderliche elektrochemische Gleichgewicht an der Elektrodenoberfläche wiederherzustellen. Zu diesem Zweck wird eine Sequenz von abwechselnd reduzierenden und oxidierenden Potentialpulsen an die Arbeitselektrode angelegt, die in Figur 2 mit CO1, CR1, CO2, CR2, CO3 und CR3 bezeichnet sind.

Vorzugsweise besteht die Sequenz der Konditionierungspulse - wie dargestellt - aus einer abwechselnden Folge oxidierender und reduzierende Potentialpulse, wobei eine gerade Gesamtzahl der reduzierenden und oxidierenden Potentialpulse besonders bevorzugt ist. Die Dauer jedes Konditionierungspulses liegt in der Praxis bei weniger als einer Sekunde, wobei Werte von weniger als 0,7 Sekunden und mehr als 0,1 Sekunden bevorzugt sind und sich der Bereich zwischen etwa 0,3 Sekunden und etwa 0,5 Sekunden besonders bewährt hat.

Für die Erfindung ist charakteristisch, daß im Anschluß an den Konditionierungsschritt 22 und vor dem in Figur 2 mit tp bezeichneten Zeitpunkt, zu dem die Probe aus dem Probenbehälter 14 über das Ventil 10 in den Durchflußkanal 3 gepumpt wird (somit die in der Probenflüssigkeit enthaltenen Beads in Kontakt mit der Arbeitselektrode 4 gebracht werden), ein zusätzlicher Potentialpuls in den Spannungsverlauf des Detektionszyklus eingefügt wird, der in Figur 2 mit DIP für "deposition improvement pulse" bezeichnet ist. Dieser zusätzliche Potentialpuls ist so hoch, daß er für eine sehr kurze, jedoch zur elektrochemischen Beeinflussung der Elektrodenoberfläche wirksame Zeitdauer (vorzugsweise mindestens etwa 0,05 Sekunden, besonders bevorzugt mindestens 0,1 Sekunden) einen oxidierenden oder reduzierenden Potentialwert erreicht. Weiter ist wesentlich, daß das Potential vor dem Zeitpunkt tp auf einen neutralen (weder oxidierenden noch reduzierenden) Potentialwert zurückgeführt wird. Besonders bevorzugt ist der dargestellte Fall eines oxidierenden DIP, wobei dieser wiederum vorzugsweise einem vorausgehenden reduzierenden Potentialpuls CR des Konditionierungsschrittes 22 folgt.

Bevorzugt hat der zusätzliche Potentialpuls DIP die gleiche Polarität wie der Potentialverlauf, der die ECL-Reaktion während des Meßschrittes 21 triggert, wobei der höchste Wert des zusätzlichen Potentialpulses DIP niedriger als der höchste Wert des die ECL-Reaktion triggernden Potentialverlaufes ist.

Im Rahmen der Erfindung wurde festgestellt, daß es mittels des DIP möglich ist, die Oberfläche der Elektrode optimal für den Abfangschritt vorzubereiten und gleichzeitig auf die Eigenschaften der Beads (z.B. Oberflächeneigenschaften, Zeta-Potential, Klebrigkeit etc.) abzustimmen. Durch experimentelle Optimierung des DIP kann das Depositionsmuster so eingestellt werden, daß es optimal den Anforderungen eines ECL-Nachweisverfahrens entspricht.

Bei Verwendung einer Platin-Arbeitselektrode entspricht der höchste Wert des zusätzlichen Potentialpulses DIP vorzugsweise einer Spannung von mindestens 0,6 V, besonders bevorzugt mindestens 0,8 V, gegenüber einer Ag/AgCl-Referenzelektrode. Dabei ist es vorteilhaft, wenn der höchste Wert des zusätzlichen Potentialpulses DIP einer Spannung von höchstens 1,6 V, vorzugsweise höchstens 1,2 V, gegenüber einer Ag/AgCl-Referenzelektrode entspricht.

Zu dem Zeitpunkt tp befindet sich der Magnet 17 in der in Figur 1 dargestellten Abfangposition. Die mit der Probenflüssigkeit durch den Durchflußkanal 3 strömenden Mikropartikel werden unter Einwirkung seines Magnetfeldes auf der Oberfläche der Arbeitselektrode abgelagert. Während des Abfangschrittes 23 und auch während des nachfolgenden Waschschrittes 24 befindet sich das Potential der Arbeitselektrode bevorzugt - wie dargestellt - im neutralen Bereich. Bei vorbekannten Verfahren ist die Arbeitselektrode während dieser Schritte meist von dem Potentiostaten getrennt, befindet sich also auf keinem definierten Potential. Gemäß der WO 89/10551 soll in diesem Abschnitt des Detektionszyklus das dort beschriebene "preoperative potential", nämlich ein konstantes oxidierendes oder reduzierendes Potential, an die Arbeitselektrode 4 angelegt werden.

Abgesehen von dem Potential der Arbeitselektrode werden der Abfangschritt 23 und der Waschschritt 24 ebenso wie der nachfolgende Meßschritt 21 in der üblichen Weise durchgeführt, wobei zu dem Zeitpunkt t_{W} das Mehrwegventil 10 derartig umgeschaltet wird, daß statt der Probenflüssigkeit die Konditionierungsflüssigkeit aus dem Behälter 13 in die Leitung 8 angesaugt wird, welche zugleich als Waschflüssigkeit für die bound/free-Trennung dient.

Statt des einen zusätzlichen Potentialpulses DIP können auch mehrere zusätzliche Potentialpulse zwischen dem Konditionierungsschritt 22 und dem Abfangschritt 23 in den Spannungsverlauf des Detektionszyklus eingefügt sein, wobei vorzugsweise alle DIPs entweder oxidierend oder reduzierend sind. Die Gesamtdauer der Zeit, in der sich der eine DIP oder die mehreren DIPs im oxidierenden bzw. reduzierenden Bereich befindet, sollte in jedem Detektionszyklus mindestens 0,05 Sekunden, vorzugsweise mindestens 0,1 Sekunden und höchstens eine Sekunde, bevorzugt höchstens 0,3 Sekunden, betragen.

Figur 3 zeigt ein zyklisches Voltamogramm einer Platinelektrode. Derartige Messungen werden üblicherweise durchgeführt, indem man das an der Elektrode anliegende Potential gegenüber einer Referenzelektrode dreieckförmig variiert und dabei die Strom/Spannung-Kurven in der dargestellten Weise registriert. Das hier dargestellte Cyclovoltamogramm resultiert aus der Messung einer Platinelektrode in Kontakt mit einer 1M-Schwefelsäurelösung. Die auf der Abszisse angegebenen Spannungswerte beziehen sich auf eine Normal-Wasserstoffelektrode. Auf der Ordinate ist der Stromfluß I in mA/cm² dargestellt.

Verfolgt man den Strom-Spannungsverlauf ausgehend von dem Punkt A in Richtung des Pfeiles, so befindet sich das Potential zunächst in einem Bereich, in dem nur ein kaum meßbarer Strom fließt, der durch die Aufladung einer Doppelschicht verursacht ist. Dieser Bereich wird in der englischsprachigen Literatur als "double-layer region" bezeichnet. In dem Kurvenabschnitt B steigt der Stromfluß stark an. Hier wird ein im Sinne der vorliegenden Erfindung oxidierender Potentialwert erreicht, d.h. die Platinoberfläche wird elektrochemisch oxidiert. Die Fläche unter der Kurve entspricht der für die Oxidation verbrauchte Ladungsmenge.

Wenn die an der Elektrode anliegende Spannung nach Erreichen ihres Maximalwertes (hier etwa 1,5 V) sinkt, ist der Stromfluß zunächst wieder gering, steigt dann jedoch in dem Bereich, in dem die Oxidschicht abgebaut wird, wieder an (Kurvenbereich C). Nach Abbau der Oxidschicht fällt die Stromstärke in dem Doppelschichtbereich wieder nahe Null ab, bis die Spannung einen Wert erreicht, bei dem eine Reduktion des (zuvor im wesentlichen reinen) Platin einsetzt. Dieser Anstieg im Kurvenbereich D markiert einen reduzierenden Potentialwert im Sinne der vorliegenden Erfindung. Der Spannungsbereich zwischen dem oxidierenden und dem reduzierenden Potential wird als neutraler Bereich N bezeichnet. Nach der erneuten Umkehrung des Potentials bei etwa 0,1 V wird die Reduktionsschicht auf der Platinoberfläche in dem Kurvenbereich E abgebaut.

### Beispiel:

Mit einem Gerät Elecsys 2010 der Boehringer Mannheim GmbH wurden Vergleichstests durchgeführt, um die Ergebnisse mit und ohne DIP zu vergleichen. Der Detektionszyklus entsprach dabei Figur 2, wobei bei den Versuchen mit DIP ein zusätzlicher oxidierender Impuls in Rechteckform mit einer Impulshöhe von + 0,9 V und einer Impulsdauer von 0,2 sec. eingefügt wurde. Für einen PSA-Test (PSA = Prostata-spezifisches Antigen) ergaben sich dabei ohne DIP folgende Werte.

**Tabelle 1:**

| Nr. | Konz. ng/ml | N | MW | VK % |
|---|---|---|---|---|
| 1 | 0.00 | 11 | 1020.94 | 2.66 |
| 2 | 0.75 | 4 | 4957.80 | 2.00 |
| 3 | 2.97 | 2 | 16698.81 | 1.75 |
| 4 | 16.20 | 2 | 84445.07 | 2.10 |
| 5 | 75.10 | 2 | 398381.77 | 0.99 |
| 6 | 142.00 | 2 | 741512.39 | 1.62 |

Mit DIP ergaben sich folgende Werte:

**Tabelle 2:**

| Nr. | Konz. ng/ml | N | MW | VK % |
|---|---|---|---|---|
| 1 | 0.00 | 11 | 1045.33 | 1.12 |
| 2 | 0.75 | 4 | 5874.34 | 0.63 |
| 3 | 2.97 | 2 | 20930.29 | 1.06 |
| 4 | 16.20 | 2 | 107626.88 | 1.26 |
| 5 | 75.10 | 2 | 497223.62 | 0.09 |
| 6 | 142.00 | 2 | 950228.61 | 0.26 |

Darin haben die Spaltenüberschriften folgende Bedeutung:
- Konz.:: Soll-Konzentration der verwendeten Kalibrations probe
- N:: Anzahl der durchgeführten Messungen
- MW:: Mittelwert des Meßsignals in willkürlichen Ein heiten
- VK:: Variationskoeffizient des Signals in Prozent

Man erkennt, daß der DIP zu einer wesentlichen Verbesserung der Signaldynamik (Quotient aus dem höchsten und niedrigsten MW) führt. Dieser Wert liegt mit DIP bei 909 und somit um etwa 25% höher als ohne DIP (726). Außerdem ist die Präzision, die sich durch die-Höhe des Variationskoeffizient VK ausdrückt, erheblich verbessert.

Durch visuelle Beobachtung und mittels Videoaufzeichnungen während des Analysezyklus ist festzustellen, daß die Beads außerdem wesentlich stabiler abgelagert werden. Ohne DIP findet während des Waschschrittes eine Bewegung der Beads statt, die für die Meßgenauigkeit nachteilig ist, insbesondere weil sie zu Verlusten von bereits abgelagerten Beads von der Arbeitselektrode führt.

## Patentansprüche

1. Verfahren zur Analyse einer flüssigen Probe, insbesondere einer Körperflüssigkeit, bezüglich einer darin befindlichen Substanz,
mittels eines Elektrochemolumineszenz-Bindungsreaktion-Verfahrens, bei dem
eine Reaktionsfolge abläuft, die mindestens eine spezifische biochemische Bindungsreaktion einschließt und zur Bildung eines eine chemolumineszierende Markersubstanz enthaltenden für die Analyse charakteristischen Komplexes und Bindung des Komplexes an magnetische Mikropartikel führt
und in einer Meßzelle (2) mit einer Arbeitselektrode (4) zur Bestimmung der Konzentration der markierten Mikropartikel ein Detektionszyklus mit einer Sequenz folgender Schritte abläuft:
- ein Reinigungsschritt (20), bei dem an die Arbeitselektrode (4) ein zur elektrochemischen Reinigung geeignetes stark oxidierendes (C10) und/oder reduzierendes (C1R) Potential angelegt wird,
- einen Konditionierungsschritt (22), bei dem eine Sequenz von abwechselnd reduzierenden und oxidierenden Potentialpulsen (CO1, CR1, CO2, CR2 ...) an die Arbeitselektrode angelegt wird,
- ein Abfangschritt (23), bei dem die Probe mit den Mikropartikeln derartig mit der Arbeitselektrode (4) kontaktiert wird, daß sich die Mikropartikel unter Einwirkung des Magnetfeldes eines auf der von der Probe abgewandten Seite der Arbeitselektrode (4) positionierten Magneten auf einer der Probe zugewandten Ablagerungsfläche (4a) der Arbeitselektrode ablagern,
- ein Meßschritt (21); bei dem ein die ECL-Reaktion triggernder Potentialverlauf an die Arbeitselektrode angelegt und das dabei infolge der Elektrochemolumineszenz von der Markierungssubstanz ausgehende Licht gemessen wird, um die Konzentration der Markierungssubstanz an der Ablagerungsfläche (4a) der Arbeitselektrode (4) zu bestimmen,
**dadurch gekennzeichnet, daß**
zur Verbesserung der gleichmäßigen Ablagerung der Mikropartikel zwischen dem Konditionierungsschritt (22) und dem Abfangschritt (23) ein zusätzlicher Potentialpuls (DIP) mit einem oxidierenden oder reduzierenden Potential in den Spannungsverlauf des Detektionszyklus eingefügt ist, wobei der zusätzliche Potentialpuls (DIP) vor der Kontaktierung (Zeitpunkt tp) der Arbeitselektrode (4) mit der Probe auf ein neutrales, weder oxidierendes noch reduzierendes, Potential zurückgeführt wird.

2. Verfahren nach Anspruch 1, **dadurch gekennzeichnet, daß** der dem zusätzlichen Potentialpuls (DIP) vorausgehende letzte Potentialpuls des Konditionierungsschritts (22) ein reduzierender Potentialpuls (CR3) ist.

3. Verfahren nach einem der Ansprüche 1 oder 2, **dadurch gekennzeichnet, daß** der zusätzliche Potentialpuls (DIP) ein oxidierender Potentialpuls ist.

4. Verfahren nach einem der vorhergehenden Ansprüche, **dadurch gekennzeichnet, daß** in dem Konditionierungsschritt abwechselnd oxidierende (CO1, CO2 ...) und reduzierende (CR1, CR2 ...) Potentialpulse an die Arbeitselektrode (4) angelegt werden.

5. Verfahren nach Anspruch 4, **dadurch gekennzeichnet, daß** die Anzahl der reduzierenden und oxidierenden Potentialpulse gleich groß ist.

6. Verfahren nach einem der vorhergehenden Ansprüche, **dadurch gekennzeichnet, daß** der zusätzliche Potentialpuls (DIP) die gleiche Polarität wie der die ECL-Reaktion während des Meßschrittes (21) triggernde Potentialverlauf hat und der höchste Wert des zusätzlichen Potentialpulses (DIP) niedriger als der höchste Wert des die ECL-Reaktion triggernden Potentialverlaufes ist.

7. Verfahren nach einem der vorhergehenden Ansprüche, **dadurch gekennzeichnet, daß** die Arbeitselektrode eine Platinelektrode ist und der höchste Wert des zusätzlichen Potentialpulses (DIP) einer Spannung von mindestens 0,6 V, vorzugsweise mindestens 0,8 V, gegenüber einer Ag/AgCl-Referenzelektrode entspricht.

8. Verfahren nach Anspruch 7, **dadurch gekennzeichnet, daß** der höchste Wert des zusätzlichen Potentialpulses (DIP) einer Spannung von höchstens 1,6 V, vorzugsweise höchstens 1,2 V, gegenüber einer Ag/AgCl-Referenzelektrode entspricht.

9. Verfahren nach einem der vorhergehenden Ansprüche, **dadurch gekennzeichnet, daß** zur Verbesserung der Ablagerung der Mikropartikel mehrere zusätzliche Potentialpulse zwischen dem Konditionierungsschritt (22) und dem Abfangschritt (23) in den Spannungsverlauf des Detektionszyklus eingefügt sind, wobei der letzte zusätzliche Potentialpuls vor der Kontaktierung der Arbeitselektrode mit der Probe auf ein neutrales, weder oxidierendes noch reduzierendes, Potential zurückgeführt wird.

10. Verfahren nach einem der vorhergehenden Ansprüche, **dadurch gekennzeichnet, daß** die Gesamtdauer der Zeit, in der sich der mindestens eine zusätzliche Potentialpuls im oxidierenden bzw. reduzierenden Potentialbereich befindet, in jedem Detektionszyklus mindestens 0,05 Sekunden, bevorzugt mindestens 0,1 Sekunden, beträgt.

11. Verfahren nach einem der vorhergehenden Ansprüche, **dadurch gekennzeichnet, daß** die Gesamtdauer der Zeit, in der sich der mindestens eine zusätzliche Potentialpuls im oxidierenden bzw. reduzierenden Potentialbereich befindet, in jedem Detektionszyklus höchstens eine Sekunde, bevorzugt höchstens 0,3 Sekunden, beträgt.

## Claims

1. Method for the analysis of a liquid sample, in particular a body liquid, with reference to a substance contained therein,
by means of an electrochemiluminescence binding reaction method wherein
a reaction sequence is carried out which includes at least one specific biochemical binding reaction and which results in the formation of a complex which contains a chemiluminescing marking substance, is characteristic for the analysis and is bound to magnetic microparticles,
and a detection cycle is carried out in a measuring cell (2) having a working electrode (4) for determination of the concentration of the marked microparticles, the detection cycle including a sequence with the following steps:
- a cleaning step (20) during which a strongly oxidizing (C1O) and/or reducing (C1R) potential is applied to the working electrode (4) for electrochemical cleaning,
- a conditioning step (22) during which a sequence of alternating reducing and oxidizing potential pulses (C01, CR1, C02, CR2...) are applied to the working electrode,
- a capturing step (23) during which the sample containing the microparticles is contacted with the working electrode (4) in such a manner that the microparticles are attracted by the magnetic field of a magnet positioned on the side of the working electrode (4) facing away from the sample and thereby deposited on a deposit surface (4a) of the working electrode facing the sample.
- a measuring step (21) during which a potential is applied to the working electrode to trigger the ECL-reaction and the electrochemiluminescense light emitted by the marking substance is measured to determine the concentration of the marking substance on the deposit surface (4a) of the working electrode (4),
**characterized in that**,
an additional potential pulse (DIP) having an oxidizing or reducing potential is introduced into the voltage curve of the detection cycle between the conditioning step (22) and the capturing step (23) for improving the even deposition of microparticles, wherein the additional potential pulse (DIP) is returned to a neutral, neither oxidizing nor reducing, potential prior to contacting (time point tₚ) of the working electrode (4) by the sample.

2. Method according to claim 1, **characterized in that** the last potential pulse of the conditioning step (22) preceding the additional potential pulse (DIP) is a reducing potential pulse (CR3).

3. Method according to any one of claims 1 or 2, **characterized in that** the additional potential pulse (DIP) is an oxidizing potential pulse.

4. Method according to any one of the preceding claims, **characterized in that** during the conditioning step alternating oxidizing (CO1, CO2,...) and reducing (CR1, CR2, ...) potential pulses are applied to the working electrode (4).

5. Method according to claim 4, **characterized in that** the numbers of the reducing and oxidizing potential pulses are equal.

6. Method according to any one of the preceding claims, **characterized in that** the additional potential pulse (DIP) has the same polarity as the potential triggering the ECL-reaction during the measuring step (21) and the highest value of the additional potential pulse (DIP) is less than the highest value of the potential triggering the ECL-reaction.

7. Method according to any one of the preceding claims, **characterized in that** the working electrode is a platinum electrode and the highest value of the additional potential pulse (DIP) corresponds to a voltage of at least 0.6 V, preferably at least 0.8 V, relative to a Ag/AgCl reference electrode.

8. Method according to claim 7, **characterized in that** the highest value of the additional potential pulse (DIP) corresponds to a voltage of at most 1.6 V, preferably at most 1.2 V, relative to an Ag/AgCl reference electrode.

9. Method according to any one of the preceding claims, **characterized in that** a plurality of additional potential pulses are introduced into the voltage curve of the detection cycle between the conditioning step (22) and the capturing step (23) for improvement of the deposition of the microparticles, wherein the last additional potential pulse is returned to a neutral, neither oxidizing nor reducing, potential prior to contacting of the working electrode by the sample.

10. Method according to any one of the preceding claims, **characterized in that** the total amount of time during which the at least one additional potential pulse is in the oxidizing or reducing potential region, respectively, is, in each detection cycle, at least 0.05 seconds and preferably at least 0.1 seconds.

11. Method according to any one of the preceding claims, **characterized in that** the total amount of time during which the at least one additional potential pulse is in the oxidizing or reducing potential region, respectively, is, in each detection cycle, at most 1 second, preferably at most 0.3 seconds.

## Revendications

1. Procédé pour l'analyse d'un échantillon liquide, en particulier un liquide corporel, concernant une substance s'y trouvant, à l'aide d'un procédé de réaction de liaison par électroluminescence, dans lequel une série de réactions se développe, qui se termine au moins par une réaction de liaison biochimique spécifique et conduit à la formation d'un complexe caractéristique pour l'analyse contenant une substance marqueur chimioluminescente et à la liaison du complexe sur des microparticules magnétiques, et dans lequel dans une cellule de mesure (2) avec une électrode de travail (4) pour la détermination de la concentration des microparticules marquées, un cycle de détection avec la séquence des étapes suivantes se développe :
- une étape de purification (20), dans laquelle on applique sur l'électrode de travail (4), un potentiel fortement oxydant (C10) et/ou réducteur (C1R) approprié pour la purification électrochimique,
- une étape de conditionnement (22), dans laquelle on applique une séquence d'impulsions de potentiels réducteurs et oxydants alternés (CO1, CR1, CO2, CR2, ...) sur l'électrode de travail,
- une étape de fixation (23), dans laquelle l'échantillon avec les microparticules est mis en contact avec l'électrode de travail (4) de sorte que les microparticules se déposent, sous l'action du champ magnétique d'un aimant positionné sur la face de l'électrode de travail (4), opposée à l'échantillon, sur une surface de dépôt (4a) de l'électrode de travail, dirigée vers l'échantillon,
- une étape de mesure (21), dans laquelle une allure de potentiel déclenchant la réaction ECL est appliquée sur l'électrode de travail et où on mesure la lumière émanant de la substance de marquage suite à l'électrochimioluminescence, pour déterminer la concentration de la substance de marquage sur la surface de dépôt (4a) de l'électrode de travail (4),
**caractérisé en ce que** pour améliorer le dépôt régulier des microparticules entre l'étape de conditionnement (22) et l'étape de fixation (23), une impulsion supplémentaire de potentiel (DIP) est ajoutée, avec un potentiel oxydant ou réducteur dans le développement de tension du cycle de détection, où l'impulsion supplémentaire de potentiel (DIP) est ramenée à un potentiel neutre, ni oxydant, ni réducteur, avant la mise en contact (moment tₚ) de l'électrode de travail (4) avec l'échantillon.

2. Procédé selon la revendication 1, **caractérisé en ce que** la dernière impulsion de potentiel de l'étape de conditionnement (22), précédant l'impulsion supplémentaire de potentiel (DIP) est une impulsion de potentiel réducteur (CR3).

3. Procédé selon la revendication 1 ou 2, **caractérisé en ce que** l'impulsion supplémentaire de potentiel (DIP) est une impulsion de potentiel oxydant.

4. Procédé selon l'une quelconque des revendications précédentes, **caractérisé en ce qu'**on applique sur l'électrode de travail, pendant l'étape de conditionnement, des impulsions de potentiels oxydants (CO1, CO2, ...) et réducteurs (CR1, CR2, ...) alternés.

5. Procédé selon la revendication 4, **caractérisé en ce que** le nombre d'impulsions de potentiels réducteurs et oxydants est de même grandeur.

6. Procédé selon l'une quelconque des revendications précédentes, **caractérisé en ce que** l'impulsion supplémentaire de potentiel (DIP) a 1 même polarité que le développement de potentiel déclenchant la réaction ECL pendant l'étape de mesure (21) et que la valeur la plus élevée de l'impulsion supplémentaire de potentiel (DIP) est plus faible que la valeur la plus élevée du développement de potentiel déclenchant la réaction ECL.

7. Procédé selon l'une quelconque des revendications précédentes, **caractérisé en ce que** l'électrode de travail est une électrode en platine et que la valeur la plus élevée de l'impulsion supplémentaire de potentiel (DIP) correspond à une tension d'au moins 0,6 V, de préférence au moins 0,8 V, par rapport à une électrode de référence Ag/AgCl.

8. Procédé selon la revendication 7, **caractérisé en ce que** la valeur la plus élevée de l'impulsion supplémentaire de potentiel (DIP) correspond à une tension d'au plus 1,6 V, de préférence au plus 1,2 V, par rapport à une électrode de référence Ag/AgCl.

9. Procédé selon l'une quelconque des revendications précédentes, **caractérisé en ce que** pour améliorer le dépôt des microparticules, on ajoute plusieurs impulsions supplémentaires de potentiel entre l'étape de conditionnement (22) et l'étape de fixation (23) dans le développement de tension du cycle de détection, où la dernière impulsion supplémentaire de potentiel est ramenée à un potentiel neutre, ni oxydant, ni réducteur, avant la mise en contact de l'électrode de travail avec l'échantillon.

10. Procédé selon l'une quelconque des revendications précédentes, **caractérisé en ce que** la durée totale de la période dans laquelle se trouve la au moins une impulsion supplémentaire de potentiel, de potentiel oxydo-réducteur, s'élève dans chaque cycle de détection, à au moins 0,05 seconde, de préférence au moins 0,1 seconde.

11. Procédé selon l'une quelconque des revendications précédentes, **caractérisé en ce que** la durée totale de la période dans laquelle se trouve la au moins une impulsion supplémentaire de potentiel, de potentiel oxydo-réducteur, s'élève dans chaque cycle de détection, à au plus 1 seconde, de préférence au plus 0,3 seconde.
